# EUROPEAN PATENT APPLICATION

(11) **EP 4 257 123 A1**
(43) Date of publication of application: **11.10.2023**
(21) Application number: 21900957.8
(22) Date of filing: 30.11.2021
(51) Int. Cl.: A61K 9/20, A61K 31/4155, A61P 19/06

(54) **STABLE ORAL FORMULATION CONTAINING 1-(3-CYANO-1-ISOPROPYL-INDOL-5-YL)PYRAZOLE-4-CARBOXYLIC ACID**

(30) Priority: 01.12.2020 KR 20200166051
(71) Applicant: Lg Chem, Ltd., Seoul 07336 (KR)
(72) Inventor: YOO, Seok Cheol, Daejeon 34122 (KR); LEE, Sun, Daejeon 34122 (KR); YUN, Duck Il, Daejeon 34122 (KR); PARK, Junghong, Daejeon 34122 (KR); SUN, Hyun Ji, Daejeon 34122 (KR)
(74) Representative: Hoffmann Eitle
(86) International application number: PCT/KR2021/017842
(87) International publication number: WO 2022/119269

(57) **Abstract**

The present invention relates to a stable oral formulation comprising 1-(3-cyano-1-isopropyl-indol-5-yl)pyrazol-4-carboxylic acid or a pharmaceutically acceptable salt thereof as an API.

The stable oral formulation according to the present invention has the characteristics of maintaining stability even if it does not comprise a stabilizer as an excipient, and does not comprise a stabilizer but has increased API content, and thus the convenience of administration can be increased.

## Description

### [Technical Field]

The present application claims the benefit of priority based on Korean Patent Application No. 10-2020-0166051 filed on December 1, 2020, the entire disclosure of which are incorporated herein by reference its entirely.

The present invention relates to an oral formulation which comprises 1-(3-cyano-1-isopropyl-indol-5-yl)pyrazol-4-carboxylic acid or a pharmaceutically acceptable salt thereof useful as a xanthine oxidase inhibitor that can prevent the deposition of uric acid in the body, as an active pharmaceutical ingredient (API), and does not comprise stabilizer, and to a preparation method thereof.

### [Background Art]

Xanthine oxidase is an enzyme that converts hypoxanthine to xanthine and also converts the formed xanthine to uric acid. It is known that when there is too much uric acid in the body, it causes various diseases, including gout and the like.

Gout refers to a condition in which crystals of uric acid accumulate in cartilage, ligaments, and surrounding tissues of joints, causing severe inflammation and pain, and the incidence of gout has been steadily increasing over the past 40 years.

Therefore, substances that inhibit the activity of xanthine oxidase can effectively treat xanthine oxidase-related diseases such as hyperuricacidemia, gout, heart failure, cardiovascular diseases, high blood pressure, diabetes, kidney diseases, inflammation, joint diseases, and inflammatory bowel disease.

Meanwhile, with respect to a substance that inhibits the activity of xanthine oxidase, KR 10-1751325 (Patent Document 1) provides 1-(3-cyano-1-isopropyl-indol-5-yl)pyrazol-4-carboxylic acid, which is a compound having the structure of Formula 1 below, and a method for preparing the compound, and KR 10-1424013 (Patent Document 2) provides various types of crystalline forms obtained by using various solvents, and a preparation method thereof.

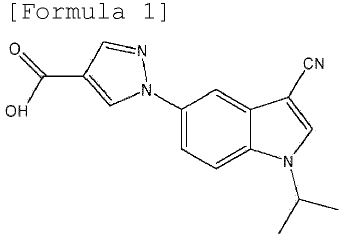

In the case of pharmaceutical formulations, it is common to comprise one or more excipients selected from diluent, disintegrant, stabilizer, binder, lubricant and glidant in addition to API which is an active ingredient. In general, excipients included in pharmaceutical formulations should be stable ingredients and should not affect the effectiveness of the formulation, but there is a problem with the stability of the API itself, or a reaction by-product between the API and a specific excipient is generated, or a reaction product that may occur depending on the combination between the excipients may affect the stability of the formulation or assay homogeneity. That is, during preparing process or storage process of the pharmaceutical formulation, problems in stability and assay homogeneity may occur due to the API itself, or degradation products or reaction products caused by contact or bonding with API and excipients.

Therefore, in pharmaceutical formulation, it is essential to properly select and combine excipients suitable for API. In addition, since the types of these excipients are very diverse and various combinations are possible, selecting a specific excipient among excipients and finding an appropriate combination to develop a stable pharmaceutical formulation is a very difficult and time-consuming step in drug development.

Since there is nothing known about the stability of an oral formulation containing Formula 1 as an API, and in the case of oral formulations, long-term storage for a period of 1 year or more is common, there is a need for improvement in securing stability and assay homoheneity of an oral formulation containing Formula 1 as an API.

### [Prior Art Documents]

### [Patent Documents]

1. KR 10- 1751325 (June 21, 2017), Novel compounds effective as xanthine oxidase inhibitors, method for preparing the same, and pharmaceutical composition containing the same
2. KR 10-1424013 (July 22, 2014), 1-(3-cyano-1-isopropyl-indol-5-yl)pyrazol-4-carboxylic acid crystalline form and the producing method thereof

### [Disclosure]

### [Technical Problem]

Accordingly, the inventors of the present invention have conducted various studies to solve the above problems, and as a result, have found through stability tests with various excipients that the use of 1-(3-cyano-1-isopropyl-indol-5-yl)pyrazol-4-carboxylic acid or a pharmaceutically acceptable salt thereof as an API does not generate decomposition products or reaction products due to contact or bonding with the excipients, and have identified an oral formulation that does not contain a stabilizer in the excipients and also has a stable effect with any combination of excipients, thereby completing the present invention.

Therefore, it is an object of the present invention to provide a stable oral formulation which comprises the API of 1-(3-cyano-1-isopropyl-indol-5-yl)pyrazol-4-carboxylic acid or a pharmaceutically acceptable salt thereof; and excipients, but does not comprise a separate stabilizer in the excipients, and a method for preparing the same.

### [Technical Solution]

The present invention provides a stable oral formulation which comprises the API of 1-(3-cyano-1-isopropyl-indol-5-yl)pyrazol-4-carboxylic acid or a pharmaceutically acceptable salt thereof and excipients, but does not comprise a separate stabilizer in the excipients.

The present invention provides a stable oral formulation which comprises the API, which is 1-(3-cyano-1-isopropyl-indol-5-yl)pyrazol-4-carboxylic acid or a pharmaceutically acceptable salt thereof, in an amount of 20% by weight or more and 60% by weight or less based on the total oral formulation; and does not comprise a stabilizer.

The oral formulation of the present invention comprises a diluent, a disintegrant, a binder, a glidant and a lubricant as excipients in addition to 1-(3-cyano-1-isopropyl-indol-5-yl)pyrazol-4-carboxylic acid or a pharmaceutically acceptable salt thereof as the API, but does not contain a stabilizer.

The oral formulation of the present invention is used for the treatment or prevention of xanthine oxidase-related diseases selected from the group consisting of hyperuricacidemia, gout, heart failure, cardiovascular diseases, high blood pressure, diabetes, kidney diseases, inflammation, joint diseases and inflammatory bowel disease.

### [Advantageous Effects]

The oral formulation comprising 1-(3-cyano-1-isopropyl-indol-5-yl)pyrazol-4-carboxylic acid or a pharmaceutically acceptable salt thereof as an active pharmaceutical ingredient (API) according to the present invention can maintain or improve stability and assay homogeneity even in any combination with excipients without containing a stabilizer in the excipients.

Accordingly, the oral formulation of the present invention provides excellent storage stability regardless of storage conditions without containing a stabilizer in the excipients.

In addition, the oral formulation according to the present invention contains a high content of 1-(3-cyano-1-isopropyl-indol-5-yl)pyrazol-4-carboxylic acid or a pharmaceutically acceptable salt thereof, which is an API, by not containing a stabilizer, and thus is not only economical, but also can increase the convenience of administration.

### [Best Mode]

Hereinafter, the present invention will be described in more detail.

All technical terms used in the present invention, unless otherwise defined, are used in the same meaning as commonly understood by those of ordinary skill in the related field of the present invention. In addition, although preferred methods and samples are described herein, similar or equivalent ones are also included in the scope of the present invention. The disclosure of all publications incorporated herein by reference are hereby incorporated by reference in their entirety.

The inventors of the present invention have continued research on oral formulations that maintain the stability of the API, by various methods, in order to develop a stable oral formulation, which comprises 1-(3-cyano-1-isopropyl-indol-5-yl)pyrazol-4-carboxylic acid or a pharmaceutically acceptable salt thereof as API, and pharmaceutically acceptable excipients, and as a result, have developed an oral formulation with excellent stability even without a stabilizer by maintaining stability and assay homogeneity , even in any combination with excipients.

In this case, since it does not contain a stabilizer to be contained in the oral formulation, the content of the API can be increased, thereby making it possible to create a high-content oral formulation with increased convenience of administration without increasing the size of the oral formulation. And it also has the additional effect of being economical compared to formulations containing other stabilizing agents.

Therefore, the present invention provides an oral formulation containing a high content of API of 1-(3-cyano-1-isopropyl-indol-5-yl)pyrazol-4-carboxylic acid or a pharmaceutically acceptable salt thereof, but not containing a stabilizer in excipients.

As used herein, term "pharmaceutically acceptable salt" refers to a salt form of a compound that does not cause serious irritation to the organism to which the compound is administered and does not impair the biological activities and physical properties of the compound. 1-(3-cyano-1-isopropyl-indol-5-yl)pyrazol-4-carboxylic acid, which is the API contained in the oral formulation of the present invention, can be converted to its salt by conventional methods.

In the oral formulation, it is common knowledge for those of ordinary skill in the pharmaceutical arts that when the API comes into contact with excipients, especially disintegrants, degradation products or reaction products of the API may be generated, thereby inhibiting the stability of the formulation, and thus, the assay of the API may be reduced. Therefore, in order to increase the stability of the API to maintain the assay homogeneity of the API, it is common knowledge for those of ordinary skill in the pharmaceutical arts that a stabilizer, which is any pharmaceutically acceptable additives (excipients), is contained in the formulation to inhibit the formation of decomposition products that may be generated when the API is mixed and contacted with additives such as excipients.

In the present invention, in order to inhibit the generation of decomposition products or reaction products by contact of 1-(3-cyano-1-isopropyl-indol-5-yl)pyrazol-4-carboxylic acid or a pharmaceutically acceptable salt thereof, which is the API, with excipients, and thus ensure stability upon oral administration, a study was conducted on oral formulations containing various excipients and combinations of excipients.

As a result, it has been confirmed that even if a stabilizer is not included as an excipient, it is possible to secure stability during oral administration and at the same time ensure assay homogeneity by inhibiting degradation products or reaction products of API that may occur during the preparing or storage process of the formulation, and an oral formulation with excellent stability without including the stabilizer was developed.

Therefore, one aspect of the present invention provides an oral formulation i) containing 1-(3-cyano-1-isopropyl-indol-5-yl)pyrazol-4-carboxylic acid or a pharmaceutically acceptable salt thereof as an API, but ii) not containing a stabilizer as an excipient.

As used herein, term "stabilization" means inhibiting any change in the API by increasing the stability of the API, and includes suppressing the production of product-related substances (decomposition products or reaction products) that may occur during the preparing process of the formulation. Therefore, the oral formulation of the present invention is stable and/or exhibits improved stability compared to other oral formulations.

As used in the present invention, the terms "stability," "stable" and variants thereof mean that the API of 90% by weight or more relative to the total weight of the API in the formulation is maintained without decomposition for 6 months under test conditions of 40°C and 75% relative humidity, or maintained without decomposition for 12 days under test conditions of 80°C and 75% relative humidity, or maintained without decomposition for 12 days under test conditions of 80 °C and 75% relative humidity, or maintained without decomposition for 1 year under test conditions of 25°C and 60% relative humidity. At this time, the API assay measured after storage under certain conditions and for a certain period, based on the initial API assay in the formulation, is called "residual assay". In various embodiments, it means that the API of for example, 90% by weight or more, 92% by weight or more, 94% by weight or more, 95% by weight or more, 96% by weight or more, 97% by weight or more, 98% by weight or more, 99% by weight or more or 99.5% by weight or more is maintained without decomposition under the conditions above. The stability of the compound, formulation, etc. can be determined within the ability of one of ordinary skill in the art using methods generally accepted in the art. For example, the amount of the API or compound can be determined by any suitable method, such as HPLC.

The decomposition is a chemical process that consists of one or more reactions, such as oxidation, reduction or hydrolysis, which, upon decomposition of a substance, cause a chemical change to produce one or more new compounds. Such novel compounds or impurities may produce reduced and/or variable amounts of API in a given formulation, thereby reducing its efficacy and exhibiting undesirable and/or detrimental side effects to the patient.

The term "impurity" in the present invention means any novel compound which is present in the composition and/or formulation in an amount of less than 10 % by weight, less than 5 % by weight, less than 3 % by weight, less than 1 % by weight, or less than 0.5 % by weight relative to the API. Thus, the amount of change in total impurities in the formulation under the conditions and time period presented herein may also be indicative of a stable formulation, which may be determined by a suitable method, for example HPLC.

In addition, the stability can be tested under the influence of various other test conditions, including, for example,
- test conditions of 25°C and 75% relative humidity (room temperature conditions) after one year,
- test conditions of 40°C and 75% relative humidity (accelerated conditions) after 6 months, or
- test conditions of 80°C and 75% relative humidity (severe conditions) after 12 days.

Also, the stability can be measured by appearance. As used herein, "visual stability" and variations thereof are intended to mean insubstantial changes in color, integrity (e.g., unbreakable), shape, and/or size of the formulation.

As used herein, the terms "enhanced stability", "improved stability" and variants thereof means that the amount of degradation of one or more APIs in a given formulation, and/or the increase in impurities in a given formulation is less than the increase in impurities in the formulation exposed to the test conditions.

It was confirmed that in the case of the oral formulation of the present invention, when performing the pliability test under severe conditions of 80°C, even if the stabilizer is not included, the residual assay of the API is maintained at 99.5% by weight or more compared to the total weight of the API when measured after 12 days, and even under conditions of 25°C and 75% relative humidity, even if the stabilizer is not included, the residual assay of the API is maintained at 99.5% by weight or more compared to the total weight of the API when measured after one year of storage, and there was no substantial change in the color, shape, size, etc. of the formulation, and thus the formulation was excellent in stability.

In the present invention, the oral formulation further comprises one or more excipients selected from a pharmaceutically acceptable diluent, disintegrant, binder, glidant, and lubricant as an excipient.

The diluent, disintegrant, binder, glidant and lubricant may be any excipients known to be commonly used in the art. The diluent may be used in an amount of 30 to 50 % by weight, 40 to 50 % by weight, or 40 to 45 % by weight, based on the total weight of the oral formulation. The disintegrant may be used in an amount ranging from 1 to 30 % by weight, 1 to 20 % by weight, 1 to 10% % by weight or 1 to 5 % by weight, based on the total weight of the oral formulation. The binder may be used in an amount ranging from 1 to 30 % by weight, 1 to 20 % by weight, 1 to 100 % by weight, or 1 to 5 % by weight, based on the total weight of the oral formulation. The glidant may be used in an amount ranging from 0.1 to 10 % by weight, 0.3 to 5 % by weight, or 0.5 to 4 % by weight, based on the total weight of the oral formulation. The lubricant may be used in an amount ranging from 0.1 to 10 % by weight, 0.3 to 5 % by weight, or 0.5 to 4 % by weight, based on the total weight of the oral formulation.

For example, the diluent may be selected from the group consisting of microcrystalline cellulose, lactose monohydrate, anhydrous lactose, lactose, starch, mannitol, carboxymethyl cellulose, sorbitol, and combinations thereof, but is not limited thereto. The disintegrant may be selected from the group consisting of low-substituted hydroxypropyl cellulose, crospovidone, croscarmellose sodium, sodium starch glycolate, F-melt^{®}, and combinations thereof, but is not limited thereto. The binder may be selected from the group consisting of hydroxypropyl cellulose, hydroxypropyl methylcellulose, hypromellose, polyvinyl acetic acid, povidone, polyvinylpyrrolidone, copovidone, Macrogol, sodium lauryl sulfate, light anhydrous silicic acid, synthetic aluminum silicate, calcium silicate or silicate derivatives such as magnesium metasilicate aluminate, phosphate such as calcium hydrogen phosphate, carbonate such as calcium carbonate, pregelatinized starch, gums such as acacia gum, gelatin, cellulose derivatives such as ethyl cellulose, and mixtures thereof, but is not limited thereto. The glidant may be selected from the group consisting of colloidal silicon dioxide, hydrated silicon dioxide and combinations thereof, but is not limited thereto. The lubricant may be selected from the group consisting of magnesium stearate, silicon dioxide, talc, light anhydrous silicic acid, sodium stearyl fumarate, and combinations thereof, but is not limited thereto.

In one embodiment, 1-(3-cyano-1-isopropyl-indol-5-yl)pyrazol-4-carboxylic acid as API in the oral formulation may be contained in the form of crystalline granules. In one example, the crystalline granules of 1-(3-cyano-1-isopropyl-indol-5-yl)pyrazol-4-carboxylic acid may be prepared by a conventionally available method for preparing crystalline granules.

The oral formulation can be administered once a day and can be taken daily.

The content of API contained in the oral formulation may be 20 to 70 % by weight, 20 to 60 % by weight, 20 to 50 % by weight, 20 to 45 % by weight, 30 to 70 % by weight, 30 to 60 % by weight, 30 to 50 % by weight, 30 to 45 % by weight, 40 to 70 % by weight, 40 to 60 % by weight, 40 to 50 % by weight, or 40 to 45 % by weight, based on the total weight of the oral formulation.

In addition, the API may be contained in a range of, for example, 50 mg to 500 mg, 50 mg to 400 mg, 50 mg to 300 mg, 50 mg to 200 mg, 50 mg to 100 mg, 100 mg to 500 mg, 100 mg to 400 mg, 100 mg to 300 mg, 100 mg to 200 mg, 200 mg to 500 mg, 200 mg to 400 mg, 200 mg to 300 mg, 300 mg to 500 mg, or 300 mg to 400 mg per unit formulation.

In addition, the API may be contained in an amount of 50 mg, 100 mg, 150 mg, 200 mg, 300 mg, 400 mg, or 455 mg per unit formulation.

The present invention provides a method for preparing an oral formulation comprising the steps of mixing an API selected from 1-(3-cyano-1-isopropyl-indol-5-yl)pyrazol-4-carboxylic acid or a pharmaceutically acceptable salt thereof and excipients of a diluent, a disintegrant, a binder, a glidant and a lubricant, which does not contain a stabilizer; and formulating the mixture.

The oral formulation may be prepared according to any preparation method known in the art, and a tablet, which is one form of the oral formulation, may also be prepared according to any tablet preparation method known in the art.

Also, the present invention provides a method for treating or preventing human xanthine oxidase-related diseases using an oral formulation comprising the 1-(3-cyano-1-isopropyl-indol-5-yl)pyrazol-4-carboxylic acid or a pharmaceutically acceptable salt thereof as an API.

As used in the present invention, the term "human xanthine oxidase-related diseases" refers to diseases that can be treated or prevented by inhibiting human xanthine oxidase, and may be, for example, hyperuricacidemia, gout, heart failure, cardiovascular diseases, high blood pressure, diabetes, diabetes-related complications, kidney diseases, inflammation, joint diseases, inflammatory bowel disease, and the like, but is not limited thereto. Examples of the diabetes-related complications may be hyperlipidemia, arteriosclerosis, obesity, high blood pressure, retinopathy, renal failure, and the like.

The term "treatment" means stopping or delaying the progression of a disease when used for a subject showing symptoms of the disease, and the term "prevention" means stopping or delaying the symptoms of the disease when used for a subject who does not show symptoms of the disease but is at a high risk of developing the disease.

Unless otherwise indicated, it is to be understood that all numbers used in the specification and claims, whether recited or not, may be modified in all instances by the term "about." It is also to be understood that the precise numbers used in the specification and claims form further embodiments of the present disclosure. Efforts have been made to ensure the accuracy of the numerical values disclosed in Examples. However, all measured values may inherently contain certain error values generated from the standard deviations measured by their respective measurement techniques.

### < Example and Experimental Example>

Hereinafter, one or more specific embodiments will be described in more detail through the following Examples and Experimental Examples. However, these Examples are only for illustrative purposes for the embodiments, and the scope of the present invention is not limited thereto.

### Experimental Example 1. Stability analysis when mixing API, 1-(3-cyano-1-isopropyl-indol-5-yl)pyrazol-4-carboxylic acid, with each excipient

As disclosed in Table 1 below, after preparing a mixture by mixing API, 1-(3-cyano-1-isopropyl-indol-5-yl)pyrazol-4-carboxylic acid, with various types of excipients such as microcrystalline cellulose, the residual assay of API at the initial stage and after 1 month, 2 months, and 3 months storage, respectively, under accelerated conditions (40°C, 75% RH; relative humidity) were measured through the analysis method and process described below. Table 1 lists only the residual assay at the initial stage and after 3 months storage.

**Table 1:**

| Item | Function | Ingredient | API:exci pient ratio (w/w) | Initial (%) | 3 Months (%) |
|---|---|---|---|---|---|
| Comparat ive Example 1 | API | 1-(3-cyano-1-isopropyl-indol-5-yl)pyrazol-4-carboxylic acid | 1:0 | 98.7 | 98.7 |
| Example 1 | Diluent | Microcrystalline cellulose (MCC101) | 1:1 | 98.8 | 98.6 |
| Example 2 | Diluent | Lactose monohydrate | 1:1 | 98.7 | 98.6 |
| Example 3 | Diluent | Anhydrous lactose | 1:1 | 98.8 | 98.6 |
| Example 4 | Diluent | Pregelatinized starch (Starch 1500^{®}) | 1:1 | 98.8 | 98.6 |
| Example 5 | Disinteg rant | Sodium starch glycolate (EXPLOTAP^{®}) | 1: 1 | 98.7 | 98.6 |
| Example 6 | Disinteg rant | Croscarmellose Sodium; (AcDisol^{®}) | 1:1 | 98.8 | 98.7 |
| Example 7 | Disinteg rant | Crospovidone | 1:1 | 98.8 | 98.6 |
| Example 8 | Disinteg rant | Low-substituted hydroxypropyl cellulose(L-HPC) | 1:1 | 98.8 | 98.6 |
| Example 9 | Binder | Povidone (PVP K-30) | 1:1 | 98.7 | 98.5 |
| Example 10 | Binder | Hypromellose (HPMC) | 1:1 | 98.7 | 98.5 |
| Example 11 | Glidant | Colloidal silicon dioxide (Aerosil^{®}) | 1:1 | 98.7 | 98.5 |
| Example 12 | Glidant | Hydrated silicon dioxide | 1:1 | 98.7 | 98.5 |
| Example 13 | Glidant | Talc | 1:1 | 98.8 | 98.5 |
| Example 14 | Lubrican t | Magnesium stearate | 1:1 | 98.7 | 98.6 |
| Example 15 | Lubrican t | Sodium stearyl fumarate (Pruv^{®}) | 1:1 | 98.8 | 98.2 |

### <Analysis method and process>

HPLC analysis conditions are as follows:
Column : Inertsil ODS-2 (4.6 x 150 mm, 5 *µ*m, GL Science)
Detector : 254 nm, UV
Flow Rate : 1.0 mℓ/min
Injection Volume : 5 *µ*ℓ
Column Temperature : 40°C

### Eluent

A : Acetonitril/Purified water/TFA(Trifluoroacetic acid) =40/60/0.1(v/v/v)
B : Acetonitril/Purified water/TFA=80/20/0.1(v/v/v)

**Table 2: Gradient Table**

| Time (min) | A (%) | B (%) |
|---|---|---|
| Initial | 100 | 0 |
| 9 | 100 | 0 |
| 15 | 0 | 100 |
| 20 | 100 | 0 |
| 30 | 100 | 0 |

According to the analysis results for stability in Table 1, it can be seen that when 1-(3-cyano-1-isopropyl-indol-5-yl)pyrazol-4-carboxylic acid alone is stored under accelerated conditions, excellent stability is shown even after 3 months (Comparative Example 1). In addition, it can be seen that even when 1-(3-cyano-1-isopropyl-indol-5-yl)pyrazol-4-carboxylic acid is mixed with various excipients and stored under accelerated conditions, it has excellent stability like 1-(3-cyano-1-isopropyl-indol-5-yl)pyrazol-4-carboxylic acid alone (Examples 1 to 15).

### Experimental Example 2. Stability analysis of tablets prepared by mixing and tableting 1-(3-cyano-1-isopropyl-indol-5-yl)pyrazol-4-carboxylic acid, which is an API, with each excipient

As disclosed in Table 3 below, 1-(3-cyano-1-isopropyl-indol-5-yl)pyrazol-4-carboxylic acid, which is an API, was mixed with various types of excipients such as microcrystalline cellulose, and then finally tableted using a tablet machine to prepare oral tablets(uncoated tablet) of Comparative Example 2 and Examples 16 to 27.

In each of the prepared oral tablets, the residual assay of the API was measured through the methods and processes described above at the initial stage of storage under severe conditions (80 °C, 75% RH) and after 12 days of severe conditions.

**Table 3:**

| Item | Function | Ingredient | API:exci pient ratio (w/w) | Initial (%) | 12 Days (%) |
|---|---|---|---|---|---|
| Comparat ive Example 2 | API | 1-(3-cyano-1-isopropyl-indol-5-yl)pyrazol-4-carboxylic acid | 1:0 | 99.80 | 99.80 |
| Example 16 | Diluent | Microcrystall ine cellulose (MCC101) | 1:1 | 99.79 | 99.8 |
| Example 17 | Diluent | Lactose monohydrate | 1:1 | 99.78 | 99.79 |
| Example 18 | Diluent | Pregelatinize d starch (Starch 1500^{®}) | 1:1 | 99.78 | 99.78 |
| Example 19 | Disintegrant | Croscarmellos e sodium (AcDisol^{®}) | 1:10 | 99.81 | 99.78 |
| Example 20 | Disintegrant | Sodium starch glycolate (EXPLOTAB^{®}) | 1: 10 | 99.80 | 99.78 |
| Example 21 | Disintegrant | Crospovidone | 1:10 | 99.81 | 99.78 |
| Example 22 | Binder | Copovidone | 1:10 | 99.81 | 99.78 |
| Example 23 | Binder | Hydroxypropyl cellulose (HPC-L) | 1:10 | 99.79 | 99.8 |
| Example 24 | Binder | Hypromellose (HPMC) | 1:10 | 99.78 | 99.79 |
| Example 25 | Binder | Hypromellose acetate succinate (HPMCAS) | 1: 10 | 99.78 | 99.78 |
| Example 26 | Binder | Povidone (PVP K-30) | 1:10 | 99.8 | 99.79 |
| Example 27 | Lubricant | Magnesium stearate | 1:10 | 98.79 | 99.8 |

According to the stability analysis results in Table 3, it can be seen that when a tablet of 1-(3-cyano-1-isopropyl-indol-5-yl)pyrazol-4-carboxylic acid alone is stored under severe conditions, the tablet shows excellent stability even after 12 days(Comparative Example 2). In addition, it can be seen that even when tablets prepared by mixing various kinds of excipients with 1-(3-cyano-1-isopropyl-indol-5-yl)pyrazol-4-carboxylic acid are stored in severe conditions, the tablets show excellent stability, as for 1-(3-cyano-1-isopropyl-indol-5-yl)pyrazol-4-carboxylic acid alone (Examples 16 to 27) .

It was confirmed that in terms of appearance, there is no substantial change in the color, shape, size, etc. of the tablet, and the tablet has excellent stability.

### Experimental Example 3: Stability analysis of mixtures of 1-(3-cyano-1-isopropyl-indol-5-yl)pyrazol-4-carboxylic acid, which is an API, with excipients

As disclosed in Table 4 below, 1-(3-cyano-1-isopropyl-indol-5-yl)pyrazol-4-carboxylic acid, which is an API, was mixed with various types of excipients such as microcrystalline cellulose to prepare mixtures of Examples 28 to 47.

In each of the prepared mixtures, the residual assay of the API was measured through the methods and processes described above at the initial stage of storage under severe conditions (80 °C, 75% RH) and after 12 days of severe conditions.

**Table 4:**

| Item | Function | Ingredient | API:exci pient ratio (w/w) | Initial (%) | 12 Days (%) |
|---|---|---|---|---|---|
| Example 28 | Diluent | Mannitol | 1:1 | 99.90 | 99.91 |
| Example 29 | Diluent | Dibasic calcium phosphate, dihydrate | 1:1 | 99.90 | 99.87 |
| Example 30 | Diluent | Dibasic calcium phosphate, anhydrous | 1:1 | 99.90 | 99.89 |
| Example 31 | Diluent | Silicified starch; (StarTab^{®}) | 1:1 | 99.90 | 99.89 |
| Example 32 | Diluent | Powdered cellulose | 1:1 | 99.90 | 99.91 |
| Example 33 | Disintegrant | Alginic acid | 1:1 | 99.90 | 99.88 |
| Example 34 | Disintegrant | Carboxymethyl cellulose calcium | 1:1 | 99.90 | 99.91 |
| Example 35 | Binder | Carboxymethyl cellulose sodium | 1:1 | 99.90 | 99.92 |
| Example 36 | Binder | Gelatin | 1:1 | 99.90 | 99.88 |
| Example 37 | Lubricant | Glyceryl behenate | 1:1 | 99.90 | 99.85 |
| Example 38 | Lubricant | Glyceryl palmitosteara te | 1:1 | 99.90 | 99.86 |
| Example 39 | Lubricant | Stearic acid | 1:1 | 99.90 | 99.86 |
| Example 40 | Lubricant | Vegetable oil, hydrogenated | 1:1 | 99.90 | 99.87 |
| Example 41 | Lubricant | Zinc stearate | 1:1 | 99.90 | 99.85 |
| Example 42 | Lubricant | Calcium stearate | 1:1 | 99.90 | 99.91 |
| Example 43 | Coating agent | Hydroxypropyl methylcellulo | 1:1 | 99.90 | 99.83 |
| | | se phthalate (HPMCP) | | | |
| Example 44 | Coating agent | Polymethacryl ates (Eudragit^{®} E PO) | 1:1 | 99.90 | 99.81 |
| Example 45 | Coating agent | Polymethacryl ates (Eudragit^{®} L 100) | 1:1 | 99.90 | 99.84 |
| Example 46 | Coating agent | Opadry II^{®} | 1:1 | 99.89 | 99.85 |
| Example 47 | Coating agent | Opadry QX^{®} | 1:1 | 99.90 | 99.84 |

According to the stability analysis results in Table 4, it can be seen that even when 1-(3-cyano-1-isopropyl-indol-5-yl)pyrazol-4-carboxylic acid is mixed with various kinds of excipients and stored under severe conditions, the API is hardly decomposed, and the initial residual assay is almost maintained, as with the result in the accelerated conditions (Example 28 to 47).

### Experimental Example 4: Stability analysis of tablets containing 1-(3-cyano-1-isopropyl-indol-5-yl)pyrazol-4-carboxylic acid, which is an API, and each excipient

When a mixture of API and an excipient such as diluent, disintegrant, binder, is formulated into an oral tablet that can be administered, even if each of the API and individual excipient component is stable in the raw material state, when formulated, the API is mixed with or in contact with an excipient, and thus there may be problems in stability, such as the generation of degradation products of the API. Therefore, to confirm this, oral tablets of a combination of API and various excipients were prepared, and stability was analyzed (The mass per tablet is 220 mg, and the content ratio of each component listed in Table 5 is based on one tablet (220 mg)).

As disclosed in Table 5 below, 1-(3-cyano-1-isopropyl-indol-5-yl)pyrazol-4-carboxylic acid, which is an API, was mixed with various types of excipients such as microcrystalline cellulose and then finally tableted using a tablet machine to prepare oral tablets of Examples 48 to 51.

The prepared tablets were measured for the residual assay of the API through the methods and processes described above at the initial stage of storage and after 12 days under severe conditions (80°C, 75% RH), respectively, or at the initial stage of storage, after storage for 3 months and 6 months under accelerated conditions (40 °C ±2 °C , 75%±5% RH), respectively, or at the initial stage of storage, after long-term storage of 1 year and 2 years or more under room temperature conditions (25°C±2°C, 60%±5% RH), respectively.

**Table 5:**

| Function | Ingredient | Example 48 | Example 49 | Example 50 | Example 51 |
|---|---|---|---|---|---|
| API | 1-(3-cyano-1-isopropyl-indol-5-yl)pyrazol-4-carboxylic acid | 45.5% | 45.5% | 45.5% | 45.5% |
| Diluent | Microcrystalline cellulose | 47.6% | 43.6% | 43.6% | 43.6% |
| | Starch | - | - | - | - |
| | Lactose anhydrous | - | - | - | - |
| | Crospovidone | 4.4% | 4.5% | - | - |
| Disinteg rant | Croscarmellose sodium | - | - | 4.5% | - |
| | Sodium starch glycolate | - | - | - | 4.50 |
| Binder | Copovidone | - | 4.5% | - | - |
| | Povidone | - | - | 4.5% | - |
| | Hydroxypropyl cellulose | - | - | - | 4.5% |
| Glidant | Colloidal silicon dioxide | 0.50 | 1% | 1% | 1% |
| Lubrican t | Sodium stearyl fumarate | 20 | - | 0.8% | - |
| | Magnesium stearate | - | 0.80 | - | 0.8% |

According to the stability analysis results in Table 6 below, it can be confirmed that when 1-(3-cyano-1-isopropyl-indol-5-yl)pyrazol-4-carboxylic acid (API) and various combinations of excipients were mixed to prepare oral tablets, even if the stabilizer is not included, product-related substance substances are rarely generated even after storage for 12 days under severe conditions (80°C, 75%RH), after 6 months of storage under accelerated conditions (40°C±2°C, 75%±5% RH), or after 12 months of storage at room temperature (25°C±2°C, 60%±5% RH), and the residual assay of the API exists in a stable form that is almost maintained when compared to the initial stage. In addition, it was confirmed that even in terms of appearance, there is no substantial change in the color, shape, size, etc. of the tablet, and the stability of the tablet is excellent.

**Table 6:**

| Item | Initial | Severe condition (80 °C, 75%RH), 12 days (%) | Accelerated condition (40°C ±2 °C, 75%±5% RH), 6 months (%) | Room temperature condition (25°C±2 °C, 60%15% RH), 12 months (%) |
|---|---|---|---|---|
| Example 48 | 99.89 | 99.89 | 99.87 | 99.87 |
| Example 49 | 99.89 | 99.88 | 99.86 | 99.86 |
| Example 50 | 99.89 | 99.89 | 99.87 | 99.86 |
| Example 51 | 99.89 | 99.88 | 99.87 | 99.87 |

## Claims

1. A stable oral formulation comprising an active pharmaceutical ingredient (API), which is 1-(3-cyano-1-isopropyl-indol-5-yl)pyrazol-4-carboxylic acid or a pharmaceutically acceptable salt thereof and one or more excipients,
wherein the formulation does not include a separate stabilizer as an excipient, and
wherein 90% by weight or more of the API is maintained at 40°C and 75% relative humidity for 6 months, maintained at 80°C and 75% relative humidity for 12 days, or maintained at 25°C and 60% relative humidity for 1 year.

2. The stable oral formulation according to claim 1, wherein 95% by weight or more of the API is maintained at 40°C and 75% relative humidity for 6 months, maintained at 80°C and 75% relative humidity for 12 days, or maintained at 25°C and 60% relative humidity for 1 year.

3. The stable oral formulation according to claim 2, wherein 99% by weight or more of the API is maintained at 40°C and 75% relative humidity for 6 months, maintained at 80°C and 75% relative humidity for 12 days, or maintained at 25°C and 60% relative humidity for 1 year.

4. The stable oral formulation according to claim 3, wherein 99.5% by weight or more of the API is maintained at 40°C and 75% relative humidity for 6 months, maintained at 80°C and 75% relative humidity for 12 days, or maintained at 25°C and 60% relative humidity for 1 year.

5. The stable oral formulation according to claim 1, wherein the oral formulation comprises at least one excipient selected from a diluent, a disintegrant, a binder, a glidant and a lubricant.

6. The stable oral formulation according to claim 5, wherein the oral formulation comprises a diluent, a disintegrant, a glidant and a lubricant.

7. The stable oral formulation according to claim 1, wherein the content of the API is 20 to 70% by weight based on the total 100% by weight of the formulation.

8. The stable oral formulation according to claim 7, wherein the content of the API is 30 to 50% by weight based on the total 100% by weight of the formulation.

9. The stable oral formulation according to claim 8, wherein the content of the API is 40 to 50% by weight based on the total 100% by weight of the formulation.

10. The stable oral formulation according to claim 1, wherein the content of the API is 50 mg per formulation.

11. The stable oral formulation according to claim 1, wherein the content of the API is 100 mg per formulation.

12. The stable oral formulation according to claim 1, wherein the content of the API is 200 mg per formulation.

13. The stable oral formulation according to claim 1, wherein the content of the API is 300 mg per formulation.
